# EUROPEAN PATENT SPECIFICATION

(11) **EP 3 615 314 B1**
(45) Date of publication and mention of the grant of the patent: **19.05.2021**
(21) Application number: 18713281.6
(22) Date of filing: 30.03.2018
(51) Int. Cl.: B29C 67/00, B29C 63/00, C07C 317/00, C08K 3/00, C08G 65/40, C08G 75/20, C08G 75/23, C08K 7/00, C08L 71/00, C08L 81/06, H05B 6/00

(54) **METHOD OF MAKING A THREE-DIMENSIONAL OBJECT USING PPSU**
VERFAHREN ZUR HERSTELLUNG EINES DREIDIMENSIONALEN OBJEKTS MIT PPSU
PROCÉDÉ DE FABRICATION D'UN OBJET TRIDIMENSIONNEL À L'AIDE DE PPSU

(30) Priority: 24.04.2017 US 201762489125 P; 19.05.2017 EP 17171975
(43) Date of publication of application: 04.03.2020
(73) Proprietor: Solvay Specialty Polymers USA, LLC, Alpharetta, Georgia 30005-3914 (US)
(72) Inventor: JEOL, Stéphane, Cumming, GA 30041 (US); SINGLETARY, Nancy J., Alpharetta, GA 30004 (US); VAN, Hai, Norcross, GA 746 (US)
(74) Representative: Fiorucci, Hélène
(86) International application number: PCT/EP2018/058317
(87) International publication number: WO 2018/197156

(56) References cited:
- WO-A1-2014/037374
- WO-A1-2016/071088
- WO-A1-2016/102330
- WO-A2-2015/130401
- US-A1- 2002 017 743
- US-A1- 2004 222 561
- US-A1- 2015 028 523
- US-A1- 2015 273 769

## Description

### Related Applications

### Technical Field

The present disclosure relates to a method of making three-dimensional (3D) objects using an additive manufacturing system, wherein the 3D object is printed from a part material comprising at least one poly(biphenyl ether sulfone) (co)polymer (PPSU) having a weight average molecular weight (Mw) ranging from 48,000 to 52,000 g/mol (as determined by gel permeation chromatography (GPC) using methylene chloride as a mobile phase and polystyrene standards). In particular, the present disclosure relates to a part material incorporating such PPSU (co)polymer, for example in the form of filaments or microparticles, for use in additive manufacturing systems to print 3D objects.

### Background

Additive manufacturing systems are used to print or otherwise build 3D parts from digital representations of the 3D parts using one or more additive manufacturing techniques. Examples of commercially available additive manufacturing techniques include extrusion-based techniques, selective laser sintering, powder/binder jetting, electron-beam melting and stereolithography processes. For each of these techniques, the digital representation of the 3D part is initially sliced into multiple horizontal layers. For each sliced layer, a tool path is then generated, which provides instructions for the particular additive manufacturing system to print the given layer.

For example, in an extrusion-based additive manufacturing system, a 3D part may be printed from a digital representation of the 3D part in a layer-by-layer manner by extruding and adjoining strips of a part material. The part material is extruded through an extrusion tip carried by a print head of the system, and is deposited as a sequence of roads on a platen in an x-y plane. The extruded part material fuses to previously deposited part material, and solidifies upon a drop in temperature. The position of the print head relative to the substrate is then incremented along a z-axis (perpendicular to the x-y plane), and the process is then repeated to form a 3D part resembling the digital representation. An example of extrusion-based additive manufacturing system starting from filaments is called Fused Filament Fabrication (FFF).

As another example, in a powder-based additive manufacturing system, a powerful laser is used to locally sinter powder into a solid part. A 3D part is created by sequentially depositing a layer of powder followed by a laser pattern to sinter an image onto that layer. An example of powder-based additive manufacturing system starting from powder is called Selective Laser Sintering (SLS).

As another example yet, carbon-fiber composites 3D part can be prepared using the continuous Fiber-Reinforced Thermosplastic (FRTP) printing method. The printing is based on fused-deposition modeling (FDM) and combines fibers and resin in a nozzle.

One of the fundamental limitations associated with known additive manufacturing methods is based on the lack of identification of a polymeric material which allows obtaining a resulting 3D part with acceptable mechanical properties.

There is therefore a need for polymeric part material to be used in additive manufacturing systems, for example FFF, SLS or FRTP printing methods, which make possible the manufacture of 3D objects presenting improved set of mechanical properties (e.g. impact resistance, elongation and tensile properties).

WO16071088 (Solvay) relates to a poly(arylether sulfone) polymer [(t-PAES) polymer], as well as to a method for preparing such polymer, in which the polymer is such that it comprises terphenyl moieties according to formula (Et). This document does not described a PPSU according to the present invention with at least 50 mol% of recurring units (R_{PPSU}) of formula (K), that-is-to-say biphenyl moieties.

WO15130401 (Texas Tech University) relates to an additive manufacturing process comprising a step of fabricating a 3D object from a filament material comprising a microwave absorbing nanomaterial, e.g. carbon-nanotubes (CNT), and irradiating the filaments during or after the step of fabricating the 3D object, in order to increase the inter-bead diffusive bond strength following the post microwave irradiation treatment and/or in-situ focused microwave beam during printing. In the long list of resin materials, the filament may be made of PPSU; however, no details about such PPSU is given, notably this document does not describe the selected molecular weight of the PPSU of the present invention and the effect of this selection on the mechanical properties of the printed parts.

US2002017743 (Stratasys) relates to a process for making a 3D object by dispensing a first thermally-solidifiable material in molten form which will define the 3D object, in coordination with dispensing of a second thermally-solidifiable material in molten form which will define a support structure for the 3D object. This document is to identify support materials which are compatible with the modeling materials. The first thermoplastic resin comprising the modeling material may selected from the group consisting of a polycarbonate resin, a polyphenylsulfone resin, and a polycarbonate/acrylonitrile-butadiene-styrene resin. According to Example 3, the modeling material is Radel® R 5600 NT and the support material is a blend of 50 wt.% Radel® R 5600 NT, 25 wt.% of Udel® P 1710 NT 15 polysulfone and 25 wt.% of EMS TR 70 amorphous polyamide. Radel® R 5600 NT corresponds to a PPSU of Mw of 46,500 g/mol (Mn of 19,200 g/mol and a PDI index of 2.48), which is outside of the Mw range claimed in the present invention.

US2004222561 (Stratasys) relates to a thermoplastic material forming a modeling filament comprising a blend of polyphenylsulphone (PPSF) as a major component, and polycarbonate (PC) as a minor component, a method for building a 3D model by layered deposition modeling using the thermoplastic material, and a method for removing supports from a completed model built from the material. According to the examples, the modeling material is a polyphenylsulfone (PPSF) or a polyphenylsulfone blend. The polyphenylsulfone tested in D4 is Radel® R 5600 NT.

US2015028523 (Stratasys) generally relates to support materials comprising polyglycolic acid (PGA) polymer. D5 notably describes a method for printing a 3D part with an additive manufacturing system, comprising the step of printing layers of a support material comprising PGA, and printing layers of a part material which can comprise according to one embodiment a thermoplastic polymer selected from the group consisting of polyethersulfone polysulfone, polyphenylsulfone, polyetherimide, and mixtures thereof (claims). Example 1 describes a filament made of polysulfone (PSU) and example 2 describes a filament made of a resin according to formula 18, that-is-to-say an end-functionalized PES polymer.

### Summary of invention

An aspect of the present disclosure is directed to a method of making a three-dimensional (3D) object, using an additive manufacturing system, comprising:
- providing a part material comprising a polymeric component comprising at least one poly(biphenyl ether sulfone) (co)polymer (PPSU) having a weight average molecular weight (Mw) ranging from 48,000 to 52,000 g/mol (as determined by gel permeation chromatography (GPC) using methylene chloride as a mobile phase and polystyrene standards), and
- printing layers of the three-dimensional object from the part material.

According to an embodiment, the method also includes the extrusion of the part material, with an extrusion-based additive manufacturing system, also known as fused filament fabrication technique (FFF).

Another aspect of the disclosure is directed to a filament material comprising at least one polymeric component comprising a poly(biphenyl ether sulfone) (co)polymer (PPSU) having a weight average molecular weight (Mw) ranging from 48,000 to 52,000 g/mol (as determined by gel permeation chromatography (GPC) using methylene chloride as a mobile phase and polystyrene standards).

Another aspect yet of the present disclosure is directed to the use of the herein described part material for the manufacture of three-dimensional objects or for the manufacture of a filament for use in the manufacture of three-dimensional objects using an additive manufacturing system, for example FFF, SLS or FRTP printing methods.

The applicant has found that the selection of a poly(biphenyl ether ketone) (co)polymer (PPSU) having a weight average molecular weight (Mw) ranging from 48,000 to 52,000 g/mol, allows the manufacture of 3D objects presenting improved mechanical properties (e.g. tensile properties and impact resistance).

The 3D objects or articles obtainable by such method of manufacture can be used in a variety of final applications. Mention can be made in particular of implantable device, dental prostheses, brackets and complex shaped parts in the aerospace industry and under-the-hood parts in the automotive industry.

### Description of embodiments

The present disclosure relates to a method of making a three-dimensional (3D) object using an additive manufacturing system, such as an extrusion-based additive manufacturing system (for example FFF), a powder-based additive manufacturing system (for example SLS) or a continuous Fiber-Reinforced Thermosplastic (FRTP) printing method.

The method of the present disclosure comprises the steps of:
- providing a part material comprising a polymeric component which comprises at least one poly(biphenyl ether sulfone) (co)polymer (PPSU) having a weight average molecular weight (Mw) ranging from 48,000 to 52,000 g/mol (as determined by gel permeation chromatography (GPC) using methylene chloride as a mobile phase and polystyrene standards), and
- printing layers of the three-dimensional (3D) object from the part material.

The merit of the applicant has been to surprisingly identify a sulfone polymer, which allow the manufacture of 3D objects having a good mechanical property profile (i.e. tensile strength, tensile elongation and impact resistance). This sulfone polymer is a poly(biphenyl ether sulfone) (co)polymer (PPSU) having a weight average molecular weight (Mw) ranging from 48,000 to 52,000 g/mol, for example from 48,500 g/mol to 51,500 g/mol (as determined by the gel permeation chromatography (GPC) method herein).

It is generally known and described in the literature that materials used for FFF or FDM must have a melt viscosity as low as possible in order to be extruded in a continuous way at the extrusion temperature. Also the melt viscosity of the polymers must be low enough so that deposited filaments lay flat rather than curl up. Melt viscosity can be lowered by increasing the temperature at which the material is extruded, but a too high temperature can cause heated material to decompose and increases energy consumption.

The Applicant hereby shows that when the PPSU approaches a critical molecular weight upper limit, i.e. Mw of more than 52,000 g/mol, the printing properties diminish and it is not anymore relevant to measure the mechanical properties of the printed part. The Applicant also shows that a PPSU having a molecular weight Mw of less than 48,000 g/mol can be printed with a good quality but that quite surprisingly in view of the common general knowledge in the art (notably the fact the melt viscosity of the polymer should be as low as possible to facilitate the fusion of the extruded polymer filaments), this PPSU does not deliver the best mechanical properties (modulus of elasticity, elongation at break and impact resistance) in comparison to a PPSU having a Mw within the claimed range, between 48,000 and 52,000 g/mol.

The expressions "(co)polymer" or "polymer" are hereby used to designate homopolymers containing substantially 100 mol.% of the same recurring units and copolymers comprising at least 50 mol.% of the same recurring units, for example at least about 60 mol.%, at least about 65 mol.%, at least about 70 mol.%, at least about 75 mol.%, at least about 80 mol.%, at least about 85 mol.%, at least about 90 mol.%, at least about 95 mol.% or at least about 98 mol.%.

The expression "part material" hereby refers to a blend of material, notably polymeric compounds, intended to form at least a part of the 3D object. The part material is according to the present disclosure used as feedstocks to be used for the manufacture of 3D objects or part of 3D objects.

The method of the present disclosure indeed employs the sulfone polymer as the main element of the part material, which can for example be shaped in the form of a filament or microparticles (with a regular shape such as spheres, or with a complex shape obtained by grinding/milling of pellets), to build a 3D object (e.g. a 3D model, a 3D article or a 3D part).

In the present application:
- any description, even though described in relation to a specific embodiment, is applicable to and interchangeable with other embodiments of the present disclosure;
- where an element or component is said to be included in and/or selected from a list of recited elements or components, it should be understood that in related embodiments explicitly contemplated here, the element or component can also be any one of the individual recited elements or components, or can also be selected from a group consisting of any two or more of the explicitly listed elements or components; any element or component recited in a list of elements or components may be omitted from such list; and
- any recitation herein of numerical ranges by endpoints includes all numbers subsumed within the recited ranges as well as the endpoints of the range and equivalents.

According to an embodiment, the part material is in the form of a filament. The expression "filament" refers to a thread-like object or fiber formed of a material or blend of materials which according to the present disclosure comprises a poly(biphenyl ether sulfone) (PPSU) having a weight average molecular weight (Mw) ranging from 48,000 to 52,000 g/mol, for example from 48,500 g/mol to 51,500 g/mol (as determined by the gel permeation chromatography (GPC) method herein).

More precisely, the filament of the present invention has a cylindrical or substantially cylindrical geometry and a diameter varying between 0.5 and 5 mm ± 0.15 mm; further, filament may have a hollow geometry, or may have a core-shell geometry, with another polymeric composition, being used to form either the core or the shell.

According to another embodiment, the invention relates to the use of a part material which is in the form of microparticles or in powder form, for example having a size comprised between 1 and 200 µm, for example between 10 and 100 µm or between 20 and 80 µm, for example for being fed through a blade, a roll or an auger-pump print head.

According to an embodiment of the disclosure, the method of making a three-dimensional object using an additive manufacturing system comprises a step consisting in extruding the part material. This step may for example occurs when printing or depositing strips or layers of part material. The method of making 3D objects using an extrusion-based additive manufacturing system is also known as fused filament fabrication technique (FFF).

FFF 3D printers are, for example, commercially available from Indmatech, from Hyrel, from Roboze or from Stratasys, Inc. (under the trade name Fortus®). SLS 3D printers are, for example, available from EOS Corporation under the trade name EOSINT® P. FRTP 3D printers are, for example, available from Markforged.

### Part material

The part material employed in the method of the present disclosure comprises a polymeric component which comprises at least one poly(biphenyl ether sulfone) (co)polymer (PPSU) having a weight average molecular weight (Mw) ranging from 48,000 to 52,000 g/mol, for example from 48,500 g/mol to 51,500 g/mol (as determined by the gel permeation chromatography (GPC) method herein).

The part material of the disclosure may include other components. For example the part material may comprise at least one additive, notably at least one additive selected from the group consisting of fillers, colorants, lubricants, plasticizers, stabilizers, flame retardants, nucleating agents, flow enhancers and combinations thereof. Fillers in this context can be reinforcing or non-reinforcing in nature.

In embodiments that include fillers, the concentration of the fillers in the part material ranges from 0.1 wt.% to 30 wt.%, with respect to the total weight of the part material. Suitable fillers include calcium carbonate, magnesium carbonate, glass fibers, graphite, carbon black, carbon fibers, carbon nanofibers, graphene, graphene oxide, fullerenes, talc, wollastonite, mica, alumina, silica, titanium dioxide, kaolin, silicon carbide, zirconium tungstate, boron nitride and combinations thereof.

According to an embodiment of the present invention, the part material may comprise at least one additive selected from the group consisting of hydroxyapatite, α-tricalcium phosphate (α-TCP), β-TCP and barium sulfate (BaSO₄).

According to one embodiment, the part material of the present disclosure comprises:
- a polymeric component comprising at least one poly(biphenyl ether sulfone) (co)polymer (PPSU) having a weight average molecular weight (Mw) ranging from 48,000 to 52,000 g/mol, for example from 48,500 g/mol to 51,500 g/mol (as determined by the gel permeation chromatography (GPC) method herein), and
- from 0 to 30 wt.% of at least one additive, for example selected from the group consisting of fillers, colorants, lubricants, plasticizers, flame retardants, nucleating agents, flow enhancers and stabilizers, based on the total weight of the part material.

According to another embodiment, the part material of the present disclosure consists essentially of:
- a polymeric component comprising at least one poly(biphenyl ether sulfone) (co)polymer (PPSU) having a weight average molecular weight (Mw) ranging from 48,000 to 52,000 g/mol, for example from 48,500 g/mol to 51,500 g/mol (as determined by the gel permeation chromatography (GPC) method herein), and
- from 0 to 30 wt.%, from 0.1 to 28 wt.% or from 0.5 to 25 wt.% of at least one additive selected from the group consisting of fillers, colorants, lubricants, plasticizers, flame retardants, nucleating agents, flow enhancers and stabilizers, based on the total weight of the part material.

### Poly(biphenyl ether sulfone) (co)polymer (PPSU)

A poly(biphenyl ether sulfone) (co)polymer is a polyarylene ether sulfone which comprises a biphenyl moiety. Poly(biphenyl ether sulfone) is also known as polyphenyl sulfone (PPSU) and for example results from the condensation of 4,4'-dihydroxybiphenyl (biphenol) and 4,4'-dichlorodiphenyl sulfone.

For the purpose of the present disclosure, a poly(biphenyl ether sulfone) (co)polymer (PPSU) denotes any polymer comprising at least 50 mol. % of recurring units (R_{PPSU}) of formula (K), the mol. % being based on the total number of moles in the polymer: 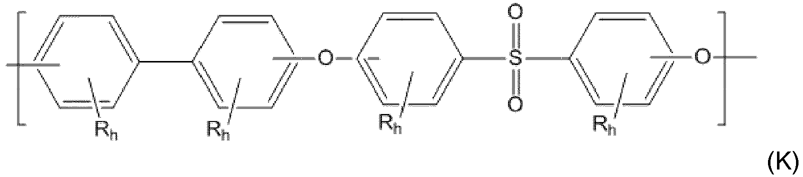 where
- R, at each location, is independently selected from the group consisting of a halogen, an alkyl, an alkenyl, an alkynyl, an aryl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine, and a quaternary ammonium; and
- h, for each R, is independently zero or an integer ranging from 1 to 4 (for example 1, 2, 3 or 4).

According to an embodiment, R is, at each location in formula (K) above, independently selected from the group consisting of a C1-C12 moiety optionally comprising one or more than one heteroatoms; sulfonic acid and sulfonate groups; phosphonic acid and phosphonate groups; amine and quaternary ammonium groups.

According to an embodiment, h is zero for each R. In other words, according to this embodiment, the recurring units (R_{PPSU}) are units of formula (K'):

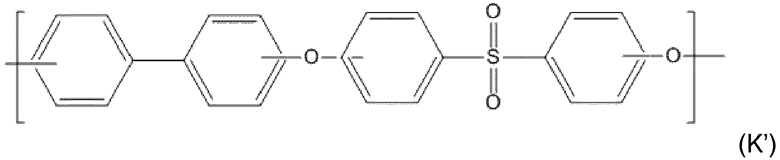

According to an embodiment of the present disclosure, at least 60 mol. %, at least 70 mol. %, at least 80 mol. %, at least 90 mol. %, at least 95 mol. %, at least 99 mol. % or all of the recurring units in the PPSU are recurring units (R_{PPSU}) of formula (K) and/or formula (K').

According to another embodiment of the present disclosure, a poly(biphenyl ether sulfone) (PPSU) denotes any polymer comprising at least 50 mol.% of recurring units (R_{PPSU}) of formula (L): 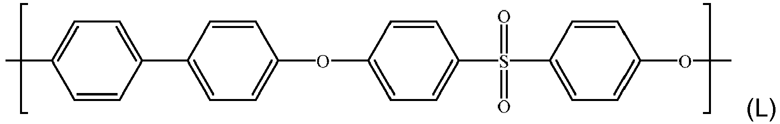 (the mol. % being based on the total number of moles in the polymer).

The PPSU polymer of the present disclosure can therefore be a homopolymer or a copolymer. If it is a copolymer, it can be a random, alternate or block copolymer.

According to an embodiment of the present disclosure, at least 60 mol. %, at least 70 mol. %, at least 80 mol. %, at least 90 mol. %, at least 95 mol. %, at least 99 mol. % or all of the recurring units in the PPSU are recurring units (R_{PPSU}) of formula (L).

When the poly(biphenyl ether sulfone) (PPSU) is a copolymer, it can be made of recurring units (R*_{PPSU}), different from recurring units (R_{PPSU}), such as recurring units of formulas (M), (N) and/or (O): 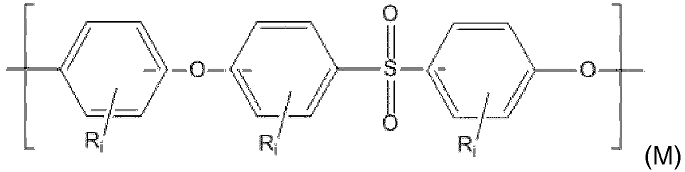 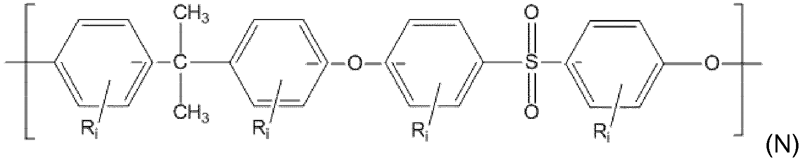 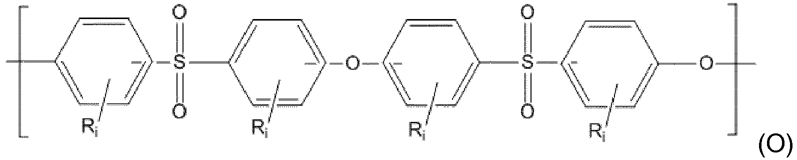 where
- R, at each location, is independently selected from a halogen, an alkyl, an alkenyl, an alkynyl, an aryl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine, and a quaternary ammonium; and
- i, for each R, is independently zero or an integer ranging from 1 to 4 (for example 1, 2, 3 or 4).

According to an embodiment, R is, at each location in formulas (M) to (O) above, independently selected from the group consisting of a C1-C12 moiety optionally comprising one or more than one heteroatoms; sulfonic acid and sulfonate groups; phosphonic acid and phosphonate groups; amine and quaternary ammonium groups.

According to an embodiment, i is zero for each R of formulas (M), (N) or (O). In other words, according to this embodiment, the recurring units (R*_{PPSU}) are units of formulas (M'), (N') and/or (O'):

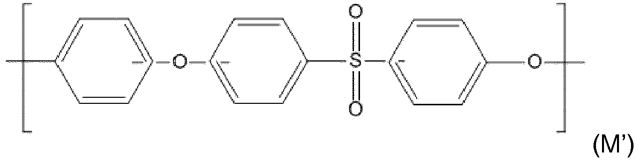

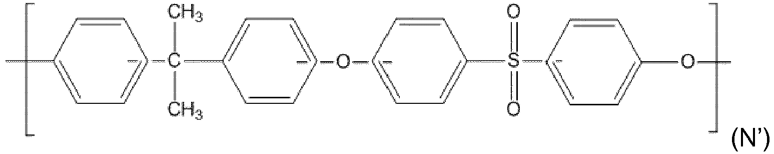

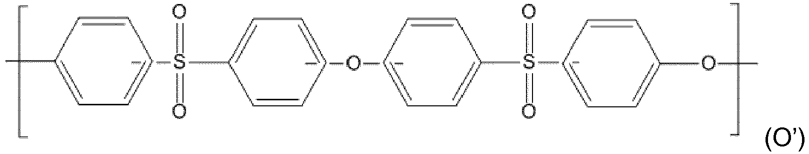

According to an embodiment of the present disclosure, less than 40 mol. %, less than 30 mol. %, less than 20 mol. %, less than 10 mol. %, less than 5 mol. %, less than 1 mol. % or all of the recurring units in the PPSU are recurring units (R*_{PPSU}) of formulas (M), (N), (O), (M'), (N') and/or (O').

According to another embodiment of the present disclosure, a poly(biphenyl ether sulfone) (PPSU) is a copolymer and has recurring units (R*_{PPSU}), different from recurring units (R_{PPSU}), such as recurring units of formulas (M"), (N") and/or (O"):

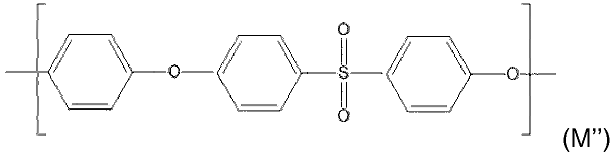

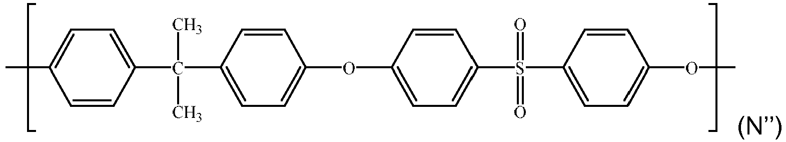

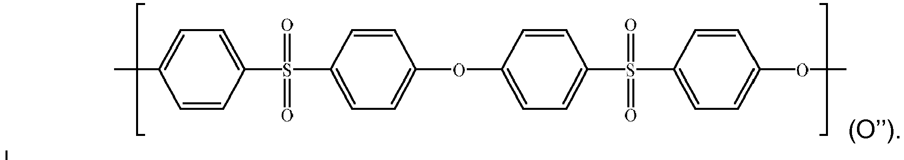

According to an embodiment of the present disclosure, less than 45 mol. %, less than 40 mol. %, less than 35 mol. %, less than 30 mol. %, less than 20 mol. %, less than 10 mol. %, less than 5 mol. %, less than 1 mol. % or all of the recurring units in the PPSU are recurring units (R*_{PPSU}) of formulas (M"), (N") and/or (O").

The poly(biphenyl ether sulfone) (PPSU) can also be a blend of a PPSU homopolymer and at least one PPSU copolymer as described above, as long as at least one of these PPSU polymer has a Mw ranging from 48,000 to 52,000 g/mol, for example from 48,500 to 51,500 g/mol or from 49,000 to 51,000 g/mol (according to the GPC method described herein). In the case of a blend of PPSU (co)polymers, the PPSU polymer having a Mw ranging from 48,000 to 52,000 g/mol in used as a major component in the polymer blend, for example in an amount of at least 50 wt.%, for example at least 55 wt.% or at least 60 wt.%, based on the total weight of the polymeric component.

The poly(biphenyl ether sulfone) (PPSU) can be prepared by any method known in the art. It can for example result from the condensation of 4,4'-dihydroxybiphenyl (biphenol) and 4,4'-dichlorodiphenyl sulfone. The reaction of monomer units takes place through nucleophilic aromatic substitution with the elimination of one unit of hydrogen halide as leaving group. It is to be noted however that the structure of the resulting poly(biphenyl ether sulfone) does not depend on the nature of the leaving group.

Defects, end groups and monomers' impurities may be incorporated in very minor amounts in the (co)polymer (PPSU) of the present disclosure, so as to advantageously not affecting negatively the performances of the same.

PPSU is commercially available as Radel® PPSU from Solvay Specialty Polymers USA, L.L.C.

According to the present disclosure, the part material comprises a polymeric component which comprises a poly(biphenyl ether sulfone) (PPSU), having a Mw ranging from 48,000 to 52,000 g/mol, for example from 48,500 to 51,500 g/mol or from 49,000 to 51,000 g/mol (according to the GPC method described herein).

According to one embodiment, the part material comprises a polymeric component which comprises at least 60 wt.% of such poly(biphenyl ether sulfone) (co)polymer(s) (PPSU), for example, at least 70 wt.%, at least 75 wt.%, at least 80 wt.%, at least 85 wt.%, at least 90 wt.%, at least 95 wt.% or at least 99 wt.%, based on the total weight of the polymeric component of the part material.

According to another embodiment, the part material comprises a polymeric component which consists essentially in a poly(biphenyl ether sulfone) (co)polymer(s) (PPSU) as defined above.

The weight average molecular weight (Mw) of the PPSU (co)polymer can be determined by gel permeation chromatography (GPC) using methylene chloride as a mobile phase, with polystyrene standards.

The polymers can be characterized by their weight average molecular weight (Mw), and they can also be characterized by their polydispersity index ("PDI" or "PDI index" herewith), also called sometimes polymolecularity index. The PDI index corresponds to the molar weight distribution of the various macromolecules within the polymer. The PDI index corresponds to the ratio Mw/Mn, Mn being the number average molecular weight and determined by GPC.

According to the present disclosure, the weight average molecular weight Mw of the PPSU polymer is from 48,000 to 52,000 g/mol, for example from 48,500 to 51,500 g/mol or from 49,000 to 51,000 g/mol.

According to another embodiment of the present disclosure, the PDI index of the PPSU polymer is from 1.8 to 2.5.

The weight average molecular weight (Mw) of the PPSU (co)polymer can be more precisely determined by gel permeation chromatography (GPC) using methylene chloride as a mobile phase and the following GPC configuration: 2x 5µ mixed D columns with guard column from Agilent Technologies ; flow rate: 1.5 mL/min; injection volume: 20 µL of a 0.2w/v% sample solution.

More precisely, the weight average molecular weight (Mw) of the PPSU (co)polymer can be measured by gel permeation chromatography (GPC), using methylene chloride as the mobile phase. In the experimental part, the following method was used: two 5µ mixed D columns with guard column from Agilent Technologies were used for separation. An ultraviolet detector of 254nm was used to obtain the chromatogram. A flow rate of 1.5ml/min and injection volume of 20 µL of a 0.2w/v% solution in mobile phase was selected. Calibration was performed with 12 narrow molecular weight polystyrene standards (Peak molecular weight range: 371,000 to 580 g/mol). The weight average molecular weight (Mw) was reported.

According to an embodiment of the present disclosure, the part material comprises:
- a polymeric component which comprises a poly(biphenyl ether sulfone) (PPSU) having a Mw ranging from 48,000 to 52,000 g/mol, for example from 48,500 to 51,500 g/mol or from 49,000 to 51,000 g/mol (according to the GPC method described herein), and
- from 0 to 30 wt.%, from 0.5 to 28 wt.% or from 1 to 25 wt.% of at least one additive selected from the group consisting of fillers, colorants, lubricants, plasticizers, flame retardants, nucleating agents, flow enhancers and stabilizers, based on the total weight of the part material.

According to another embodiment of the present disclosure, the part material comprises:
- a polymeric component which comprises a poly(biphenyl ether sulfone) (PPSU) having a Mw ranging from 48,000 to 52,000 g/mol, for example from 48,500 to 51,500 g/mol or from 49,000 to 51,000 g/mol and a PDI index from 1.8 and 2.5 (according to the GPC method described herein), and
- from 0 to 30 wt.%, from 0.5 to 28 wt.% or from 1 to 25 wt.% of at least one additive selected from the group consisting of fillers, colorants, lubricants, plasticizers, flame retardants, nucleating agents, flow enhancers and stabilizers, based on the total weight of the part material.

Such part material presents, when used to manufacture 3D objects, a good mechanical property profile (i.e. tensile strength, tensile elongation and impact resistance) over a PPSU polymer of higher or lower Mw.

The part material of the present disclosure can be made by methods well known to the person of ordinary skill in the art. For example, such methods include, but are not limited to, melt-mixing processes. Melt-mixing processes are typically carried out by heating the polymer components above the melting temperature of the thermoplastic polymers thereby forming a melt of the thermoplastic polymers. In some embodiments, the processing temperature ranges from about 280-450°C, preferably from about 290-440°C, from about 300-430°C or from about 310-420°C. Suitable melt-mixing apparatus are, for example, kneaders, Banbury mixers, single-screw extruders, and twin-screw extruders. Preferably, use is made of an extruder fitted with means for dosing all the desired components to the extruder, either to the extruder's throat or to the melt. In the process for the preparation of the part material, the components of the part material, i.e. PPSU and optionally additives, are fed to the melt-mixing apparatus and melt-mixed in that apparatus. The components may be fed simultaneously as a powder mixture or granule mixer, also known as dry-blend, or may be fed separately.

The order of combining the components during melt-mixing is not particularly limited. In one embodiment, the component can be mixed in a single batch, such that the desired amounts of each component are added together and subsequently mixed. In other embodiments, a first sub-set of components can be initially mixed together and one or more of the remaining components can be added to the mixture for further mixing. For clarity, the total desired amount of each component does not have to be mixed as a single quantity. For example, for one or more of the components, a partial quantity can be initially added and mixed and, subsequently, some or all of the remainder can be added and mixed.

### Filament material

The present disclosure also relates to a filament material comprising a polymeric component comprising at least one poly(biphenyl ether sulfone) (PPSU) having a weight average molecular weight (Mw) ranging from 48,000 to 52,000 g/mol (as determined by gel permeation chromatography (GPC) using methylene chloride as a mobile phase and polystyrene standards).

This filament material is well-suited for use in a method of making a three-dimensional object.

All of the embodiments described above with respect to the part material do apply equally to the filament material.

As an example, the filament material of the disclosure may include other components. For example the filament material may comprise at least one additive, notably at least one additive selected from the group consisting of fillers, colorants, lubricants, plasticizers, stabilizers, flame retardants, nucleating agents, flow enhancers and combinations thereof.

The filament of the present invention hasve a cylindrical or substantially cylindrical geometry and a diameter varying between 0.5 and 5 mm ± 0.15 mm; further, filament may have a hollow geometry, or may have a core-shell geometry, with the support material of the present disclosure being used to form either the core or the shell.

The diameter of the filament varies between 0.5 mm and 5 mm, for example between 0.8 and 4 mm or for example between 1 mm and 3.5 mm. The diameter of the filament can be chosen to feed a specific FFF 3D printer. An example of filament diameter used extensively in FFF process is 1.75 mm or 2.85 mm diameter.A good control of the filament size with a reduced standard deviation can be obtained with the PPSU polymer of the present invention. Notably, the filament can have a cylindrical geometry and a diameter comprised between 0.5 and 5 mm ± 0.15 mm, for example between 0.8 and 4 mm ± 0.1 mm or for example between 1 and 3.5 mm ± 0.08 mm.

The filament of the present disclosure can be made from the part material by methods including, but not limited to, melt-mixing processes. Melt-mixing processes are typically carried out by heating the polymer components above the highest melting temperature and glass transition temperature of the thermoplastic polymers thereby forming a melt of the thermoplastic polymers. In some embodiments, the processing temperature ranges from about 280-450°C, preferably from about 290-440°C, from about 300-430°C or from about 310-420°C.

The process for the preparation of the filament can be carried out in a melt-mixing apparatus, for which any melt-mixing apparatus known to the one skilled in the art of preparing polymer compositions by melt mixing can be used. Suitable melt-mixing apparatus are, for example, kneaders, Banbury mixers, single-screw extruders, and twin-screw extruders. Preferably, use is made of an extruder fitted with means for dosing all the desired components to the extruder, either to the extruder's throat or to the melt. In the process for the preparation of the filament, the components of the part material, i.e. at least PPSU and optionally additives, are fed to the melt-mixing apparatus and melt-mixed in that apparatus. The components may be fed simultaneously as a powder mixture or granule mixer, also known as dry-blend, or may be fed separately.

The order of combining the components during melt-mixing is not particularly limited. In one embodiment, the component can be mixed in a single batch, such that the desired amounts of each component are added together and subsequently mixed. In other embodiments, a first sub-set of components can be initially mixed together and one or more of the remaining components can be added to the mixture for further mixing. For clarity, the total desired amount of each component does not have to be mixed as a single quantity. For example, for one or more of the components, a partial quantity can be initially added and mixed and, subsequently, some or all of the remainder can be added and mixed.

The method of making the filaments also comprises a step of extrusion, for example with a die. For this purpose, any standard molding technique can be used; standard techniques including shaping the polymer compositions in a molten/softened form can be advantageously applied, and include notably compression molding, extrusion molding, injection molding, transfer molding and the like. Extrusion molding is preferred. Dies may be used to shape the articles, for example a die having a circular orifice if the article is a filament of cylindrical geometry.

The method may comprise if needed several successive steps of melt-mixing or extrusion under different conditions.

The process itself, or each step of the process if relevant, may also comprise a step consisting in a cooling of the molten mixture.

### Support material

The method of the present disclosure may also employ another polymeric component to support the 3D object under construction. This polymeric component, similar or distinct from the part material used to build a 3D object, is hereby called support material. Support material may be required during 3D printing to provide vertical and/or lateral support in the higher operating conditions required for the high-temperature part materials (e.g. PPSU requiring a processing temperature around 320-400° C).

The support material, possibly used in the context of the present method, advantageously possesses a high melting temperature (i.e. above 260°C), in order to resist high temperature applications. The support material may also possess a water absorption behaviour or a solubility in water at a temperature lower than 110°C, in order sufficiently swell or deform upon exposure to moisture.

According to an embodiment of the present disclosure, the method of making a three-dimensional object using an additive manufacturing system further comprises the steps of:
- providing a support material,
- printing layers of a support structure from the support material, and
- removing at least a portion of the support structure from the three-dimensional object.

A variety of polymeric components can be used as a support material. Notably, support material can comprise polyamides or copolyamides, such as for example the ones described in co-pending US provisional application # 62/316,835 and co-pending US provisional application # 62/419,035.

### Applications

The present disclosure also relates to the use of a part material comprising a polymeric component comprising at least one poly(biphenyl ether sulfone) (PPSU) having a weight average molecular weight (Mw) ranging from 48,000 to 52,000 g/mol (as determined by gel permeation chromatography (GPC) using methylene chloride as a mobile phase and polystyrene standards), for the manufacture of three-dimensional objects using an additive manufacturing system, for example FFF, SLS or FRTP printing methods.

The present disclosure also relates to the use of a filament material comprising a polymeric component comprising at least one poly(biphenyl ether sulfone) (PPSU) having a weight average molecular weight (Mw) ranging from 48,000 to 52,000 g/mol (as determined by gel permeation chromatography (GPC) using methylene chloride as a mobile phase and polystyrene standards), for the manufacture of three-dimensional objects, for example using an additive manufacturing system, for example FFF, SLS or FRTP printing methods.

All of the embodiments described above with respect to the part material do apply equally to the use of the part material or the use of the filament material.

The present disclosure also relates to the use of a part material comprising a polymeric component comprising at least one poly(biphenyl ether sulfone) (PPSU) having a weight average molecular weight (Mw) ranging from 48,000 to 52,000 g/mol (as determined by gel permeation chromatography (GPC) using methylene chloride as a mobile phase and polystyrene standards), for the manufacture of a filament- having a cylindrical geometry and a diameter varying between 0.5 and 5 mm ± 0.15 mm for use in the manufacture of three-dimensional objects, for example using an additive manufacturing system, for example FFF, SLS or FRTP printing methods.

The present disclosure also relates to 3D objects or 3D articles obtainable, at least in part, from the method of making 3D object(s) of the present disclosure, using the part material herein described. These 3D objects or 3D articles present a density comparable to injection molded objects or articles. They also present comparable or improved mechanical properties, notably impact strength (or impact resistance, for example notched impact resistance), stiffness (measured as the modulus of elasticity), tensile strength or elongation.

The 3D objects or articles obtainable by such method of making can be used in a variety of final applications. Mention can be made in particular of implantable device, dental prostheses, brackets and complex shaped parts in the aerospace industry and under-the-hood parts in the automotive industry.

### EXAMPLES

The disclosure will be now described in more detail with reference to the following examples, whose purpose is merely illustrative and not intended to limit the scope of the disclosure.

### Starting Materials

The following polymers were used to prepare filaments of examples 1 and 2:
PPSU #1: a poly(biphenyl ether sulfone) (PPSU) with a Mw of 50,500 g/mol, prepared according to the following process:
   The synthesis of the PPSU was achieved by the reaction in a 1L flask of 83.8 g of 4,4'-biphenol (0.450 mol), 131.17 g of 4,4'-dichlorodiphenyl sulfone (0.457 mol) dissolved in a mixture of 400 g of sulfolane with the addition of 66.5g (0.481 mol) of dry K₂CO₃.
   The reaction mixture was heated up to 210 °C and maintained at this temperature until the polymer had the expected Mw. An excess of methyl chloride was then added to the reaction.
   The reaction mixture was diluted with 600 g of MCB. The poly(biphenyl ether sulfone) was recovered by filtration of the salts, coagulation, washing and drying. The GPC analysis showed a number average molecular weight (Mw) of 50,500 g/mol, an average molecular weight (Mn) of 21,500 g/mol and PDI index is 2.35.
PPSU #2: a poly(biphenyl ether sulfone) (PPSU) with a Mw of 46,500 g/mol, a Mn of 19,200 g/mol and a PDI index of 2.48, prepared according to the same process than PPSU #1, except that the reaction was stopped earlier. PPSU #2 corresponds to Radel® R 5600 NT described in example 3 of US 2002/0017743 (Modeling Material).
PPSU #3: a poly(biphenyl ether sulfone) (PPSU) with a Mw of 55,000 g/mol, a Mn of 22,000 g/mol and PDI index is 2.5, prepared according to the same process than PPSU #1, except that the reaction was stopped later.

### Filament Preparation

Filaments of diameter of 1.75 ± 0.07 mm were prepared for each polymer above prepared, using a Brabender® Intelli-Torque Plasti-Corde® Torque Rheometer extruder equipped with a 0.75" 32 L/D general purpose single screw, a filament head adapter, a 2.5-mm nozzle and ESI-Extrusion Services downstream equipment comprising a cooling tank, a belt puller, and a Dual Station Coiler. A Beta LaserMike® DataPro 1000 was used to monitor filament dimensions. The melt strands were cooled with air. The Brabender® zone set point temperatures were as follows: zone 1, 350°C; zone 2, 340°C; zones 3 and 4, 330°C. The Brabender® speed ranged from 30 to 50 rpm and the puller speed from 23 to 37 fpm.

A good control of the filament size with a reduced standard deviation can be obtained with the exemplified PPSU.

### Test methods

### * Weight average molecular weight (Mw) and number average molecular weight (Mn) of the PPSU polymers

The molecular weight was measured by gel permeation chromatography (GPC), using methylene chloride as a mobile phase. Two 5µ mixed D columns with guard column from Agilent Technologies were used for separation. An ultraviolet detector of 254nm was used to obtain the chromatogram. A flow rate of 1.5 ml/min and injection volume of 20 µL of a 0.2 w/v% solution in mobile phase was selected. Calibration was performed with 12 narrow molecular weight polystyrene standards (Peak molecular weight range: 371,000 to 580 g/mol). The weight average molecular weight (Mw) and number average molecular weight (Mn) was reported.

### * Printing quality and Impact strength

Notched impact strength was determined according to the ASTM D256 method using a 2-ftlb hammer.

### *Tensile strength

Tensile strength and modulus were determined according to the ASTM D638 method with Type V bars.

The test bars (according to the present disclosure or comparative) and their mechanical properties are reported in Table 1 below (5 test bars/mean value).

### Example 1 - Printing with Indmatec® HPP 155 3D printer

### Fused Filament Fabrication bars (FFF bars)

Test bars (i.e. ASTM D638Type V bars) were printed from the above filaments of 1.75 mm in diameter on an Indmatec® HPP 155 3D printer equipped with a 0.6 mm diameter nozzle. Bars were oriented in the XY direction on the build platform during printing. Test bars were printed with a 10 mm-wide brim and three perimeters. The tool path was a cross-hatch pattern with a 45° angle with respect to the long axis of the part. The build plate temperature for all bars was 100°C. The nozzle and extruder temperature was 385°C. The speed of the nozzle was varied from 8 to 18 mm/s. The first layer height in each case was 0.3 mm, with subsequent layers deposited at 0.1 mm height and 100% fill density.

**Table 1**

| | 1 | 2 | 3 |
|---|---|---|---|
| C: comparative | I | C | C |
| I: according to the invention | | | |
| PPSU #1 - Mw: 50,500 g/mol | 100 | | |
| PPSU #2 - Mw: 46,500 g/mol | | 100 | |
| PPSU #3 - Mw: 55,000 g/mol | | | 100 |
| Process | FFF | FFF | FFF |
| Printing quality | + | + | - |
| Modulus of Elasticity (ksi) | 333 | 313 | *NR* |
| Nominal Tensile Strain at Break (%) | 17.5 | 13.2 | *NR* |
| Notched Impact (ft-lb/in) | 6.37 | 3.25 | *NR* |
| Testing Speed (in/min) | 0.05 | 0.05 | 0.05 |

### NR: non relevant

The printing quality is assessed according to the Notched Impact test and the type of break obtained therefrom:
(-) means that the sample presents inter-layer delamination;
(+) means that the sample breaks according to a pattern similar to injection molded parts.

As shown in Table 1, it was not possible to print test bars of reasonable quality by FFF using filaments of PPSU #3. PPSU #3 exceeds the critical molecular weight Mw and cannot be printed by FFF; the values of the mechanical properties of such bars are therefore non relevant (NR).

The test bars of example 1 (obtained by FFF with a filament of PPSU having a Mw of 50,500 g/mol) exhibit a modulus of elasticity, an elongation at break and an impact resistance that are higher than the test bars of example 2 (obtained by FFF with a filament of PPSU of lower Mw, 46,500 g/mol), while maintaining a good printability.

The PPSU polymer having a Mw of 50,500 g/mol is therefore well-suited to the requirements of Fused Filament Fabrication according to the present disclosure.

### Example 2 - Printing with Hyrel® Hydra 430 3D printer

### Fused Filament Fabrication bars (FFF bars)

Test bars (i.e. ASTM D638Type V bars) were printed from filaments of 1.75 mm in diameter on a Hyrel Hydra 430 3D printer equipped with a 0.5 mm diameter nozzle. Bars were oriented in the XY direction on the build platform during printing. Test bars were printed with a 10 mm-wide brim and three perimeters, according to ASTM Additive Manufacturing standard F2971-13. The tool path was a cross-hatch pattern with a 45° angle with respect to the long axis of the part. The build plate temperature for all bars was 180°C. The nozzle and extruder temperature was 385°C. The speed of the nozzle was 20 mm/s for the first layer and 40 mm/s for subsequent layers. The first layer height in each case was 0.1 mm, with subsequent layers deposited at 0.1 mm height and 100% fill density.

**Table 2**

| | 4 |
|---|---|
| I: according to the invention | I |
| PPSU #1 - Mw: 50,500 g/mol | 100 |
| Process | FFF |
| Printing quality | + |
| Nominal Tensile Strain at Break (%) | 21 |
| Tensile Strength at Yield (MPa) | 62 |
| Notched Impact (ft-lb/in) | 9.03 |
| Testing Speed (in/min) | 0.05 |

The test bars of example 4 (obtained by FFF with a filament of PPSU having a Mw of 50,500 g/mol) under different printing conditions exhibits a very good elongation and tensile strength at break, as well as an impact resistance which are similar to the performances of parts obtained by injection molding.

## Claims

1. A method of making a three-dimensional (3D) object using an additive manufacturing system, comprising:
- providing a part material comprising a polymeric component comprising at least one poly(biphenyl ether sulfone) (co)polymer (PPSU) having a weight average molecular weight (Mw) ranging from 48,000 to 52,000 g/mol (as determined by gel permeation chromatography (GPC) using methylene chloride as a mobile phase and polystyrene standards),
- printing layers of the three-dimensional object from the part material, wherein the poly(biphenyl ether sulfone) (co)polymer (PPSU) comprises at least 50 mol. % of recurring units (R_{PPSU}) of formula (K), the mol. % being based on the total number of moles in the polymer: 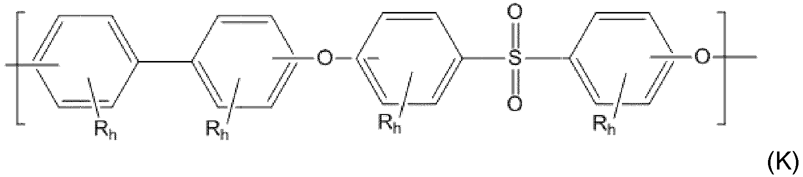 where
- R, at each location, is independently selected from a halogen, an alkyl, an alkenyl, an alkynyl, an aryl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine, and a quaternary ammonium; and
- h, for each R, is independently zero or an integer ranging from 1 to 4.

2. The method of claim 1, wherein the part material also comprises up to 30 wt.%, based on the total weight of the part material, of at least one additive selected from the group consisting of fillers, colorants, lubricants, plasticizers, flame retardants, nucleating agents, flow enhancers and stabilizers.

3. The method of claim 1 or 2, wherein the polymeric component of the part material comprises at least 80 wt. % of poly(biphenyl ether sulfone) (co)polymer(s) (PPSU), based on the total weight of polymeric component of the part material.

4. The method of any one of claims 1-3, wherein the part material is in the form of a filament or microparticles.

5. The method of anyone of claims 1-4, wherein the step of printing layers comprises extruding the part material.

6. The method of anyone of claims 1-5, wherein the poly(biphenyl ether sulfone) (co)polymer (PPSU) comprises at least 60 mol. % of recurring units (R_{PPSU}) of formula (K), the mol. % being based on the total number of moles in the polymer: 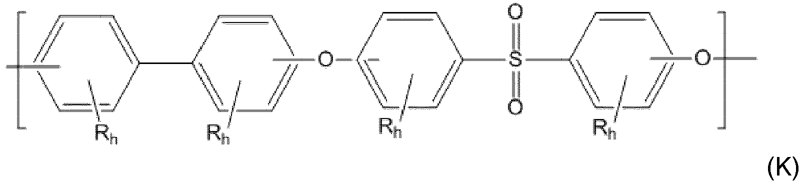 where
- R, at each location, is independently selected from a halogen, an alkyl, an alkenyl, an alkynyl, an aryl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine, and a quaternary ammonium; and
- h, for each R, is independently zero or an integer ranging from 1 to 4.

7. The method of anyone of claims 1-5, wherein the poly(biphenyl ether sulfone) (co)polymer (PPSU) comprises at least 60 mol. % of recurring units (RPPSU) of formula (L), the mol. % being based on the total number of moles in the polymer:

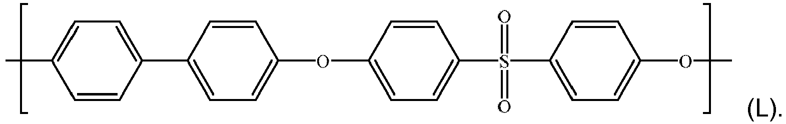

8. The method of claim 6 or 7, wherein the poly(biphenyl ether sulfone) (co)polymer (PPSU) comprises at least 90 mol. % of recurring units (R_{PPSU}) of formula (L) and/or (K), the mol. % being based on the total number of moles in the polymer.

9. A filament material having a cylindrical geometry and a diameter varying between 0.5 and 5 mm ± 0.15 mm, comprising a polymeric component comprising a poly(biphenyl ether sulfone) (co)polymer (PPSU) having a weight average molecular weight (Mw) ranging from 48,000 to 52,000 g/mol (as determined by gel permeation chromatography (GPC) using methylene chloride as a mobile phase and polystyrene standards)
wherein the poly(biphenyl ether sulfone) (co)polymer (PPSU) comprises at least 50 mol. % of recurring units (R_{PPSU}) of formula (K), the mol. % being based on the total number of moles in the polymer: 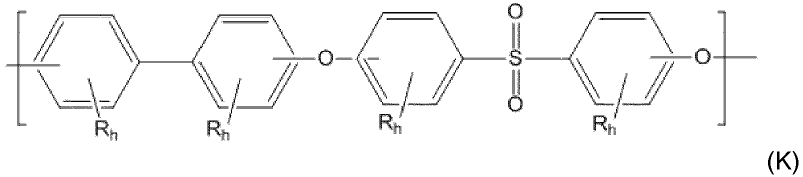 where
- R, at each location, is independently selected from a halogen, an alkyl, an alkenyl, an alkynyl, an aryl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine, and a quaternary ammonium; and
- h, for each R, is independently zero or an integer ranging from 1 to 4.

10. The filament material of claim 9, wherein the polymeric component comprises at least 80 wt. % of poly(biphenyl ether sulfone) (co)polymer (PPSU), based on the total weight of the polymeric component of the filament material.

11. Use of a part material comprising a polymeric component comprising at least one poly(biphenyl ether sulfone) (co)polymer (PPSU) having a weight average molecular weight (Mw) ranging from 48,000 to 52,000 g/mol (as determined by gel permeation chromatography (GPC) using methylene chloride as a mobile phase and polystyrene standards), for the manufacture of three-dimensional objects using an additive manufacturing system
wherein the poly(biphenyl ether sulfone) (co)polymer (PPSU) comprises at least 50 mol. % of recurring units (R_{PPSU}) of formula (K), the mol. % being based on the total number of moles in the polymer: 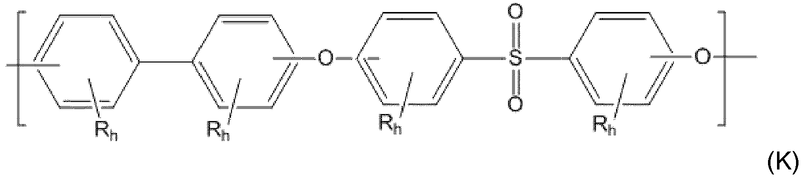 where
- R, at each location, is independently selected from a halogen, an alkyl, an alkenyl, an alkynyl, an aryl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine, and a quaternary ammonium; and
- h, for each R, is independently zero or an integer ranging from 1 to 4.

12. Use of claim 11, wherein the part material also comprises up to 30 wt.%, based on the total weight of the part material, of at least one additive selected from the group consisting of fillers, colorants, lubricants, plasticizers, flame retardants, nucleating agents, flow enhancers and stabilizers.

13. Use of anyone of claims 11-12, wherein the polymeric component of the part material comprises at least 80 wt. % of poly(biphenyl ether sulfone) (co)polymer (PPSU), based on the total weight of polymeric component of the part material.

14. Use of anyone of claims 11-13, wherein the part material is in the form of a filament or microparticles.

15. Use of a part material comprising a polymeric component comprising at least one poly(biphenyl ether sulfone) (co)polymer (PPSU) having a weight average molecular weight (Mw) ranging from 48,000 to 52,000 g/mol (as determined by gel permeation chromatography (GPC) using methylene chloride as a mobile phase and polystyrene standards), for the manufacture of a filament having a cylindrical geometry and a diameter varying between 0.5 and 5 mm ± 0.15 mm for use in the manufacture of three-dimensional objects wherein the poly(biphenyl ether sulfone) (co)polymer (PPSU) comprises at least 50 mol. % of recurring units (R_{PPSU}) of formula (K), the mol. % being based on the total number of moles in the polymer: 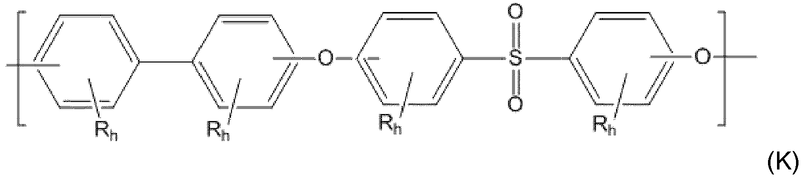 where
- R, at each location, is independently selected from a halogen, an alkyl, an alkenyl, an alkynyl, an aryl, an ether, a thioether, a carboxylic acid, an ester, an amide, an imide, an alkali or alkaline earth metal sulfonate, an alkyl sulfonate, an alkali or alkaline earth metal phosphonate, an alkyl phosphonate, an amine, and a quaternary ammonium; and
- h, for each R, is independently zero or an integer ranging from 1 to 4.

## Patentansprüche

1. Verfahren zur Herstellung eines dreidimensionalen (3D) Gegenstands unter Verwendung eines additiven Fertigungssystems, umfassend:
- Bereitstellen eines Teilematerials, das eine Polymerkomponente umfasst, die wenigstens ein Poly(biphenylethersulfon)-(Co)polymer (PPSU) mit einem gewichtsgemittelten Molekulargewicht (Mw) in dem Bereich von 48.000 bis 52.000 g/mol (wie bestimmt durch Gelpermeationschromatographie (GPC) unter Verwendung von Methylenchlorid als mobile Phase und Polystyrolstandards) umfasst,
- Drucken von Schichten des dreidimensionalen Gegenstands aus dem Teilematerial, wobei das Poly(biphenylethersulfon)-(Co)polymer (PPSU) wenigstens 50 mol-% Wiederholungseinheiten (R_{PPSU}) der Formel (K) umfasst, wobei die mol-% auf die Gesamtzahl von Molen in dem Polymer bezogen sind: 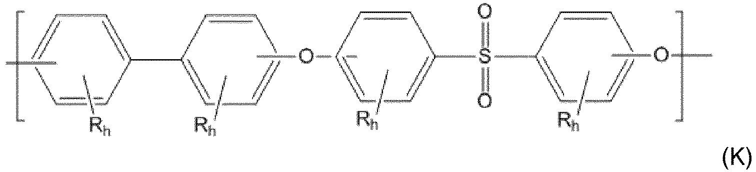 wobei
- R an jeder Stelle unabhängig ausgewählt ist aus einem Halogen, einem Alkyl, einem Alkenyl, einem Alkinyl, einem Aryl, einem Ether, einem Thioether, einer Carbonsäure, einem Ester, einem Amid, einem Imid, einem Alkali- oder Erdalkalimetallsulfonat, einem Alkylsulfonat, einem Alkali- oder Erdalkalimetallphosphonat, einem Alkylphosphonat, einem Amin und einem quaternären Ammonium; und
- h für jedes R unabhängig null oder eine ganze Zahl in dem Bereich von 1 bis 4 ist.

2. Verfahren gemäß Anspruch 1, wobei das Teilematerial ferner bis zu 30 Gew.-%, bezogen auf das Gesamtgewicht des Teilematerials, an wenigstens einem Zusatzstoff ausgewählt aus der Gruppe bestehend aus Füllstoffen, Farbstoffen, Schmiermitteln, Weichmachern, Flammhemmern, Keimbildnern, Fließverbesserern und Stabilisatoren umfasst.

3. Verfahren gemäß Anspruch 1 oder 2, wobei die Polymerkomponente des Teilematerials wenigstens 80 Gew.-% Poly(biphenylethersulfon)-(Co)polymer(e) (PPSU), bezogen auf das Gesamtgewicht der Polymerkomponente des Teilematerials, umfasst.

4. Verfahren gemäß einem der Ansprüche 1-3, wobei das Teilematerial in der Form eines Filaments oder von Mikropartikeln vorliegt.

5. Verfahren gemäß einem der Ansprüche 1-4, wobei der Schritt des Druckens von Schichten Extrudieren des Teilematerials umfasst.

6. Verfahren gemäß einem der Ansprüche 1-5, wobei das Poly(biphenylethersulfon)-(Co)polymer (PPSU) wenigstens 60 mol-% Wiederholungseinheiten (R_{PPSU}) der Formel (K) umfasst, wobei die mol-% auf die Gesamtzahl von Molen in dem Polymer bezogen sind: 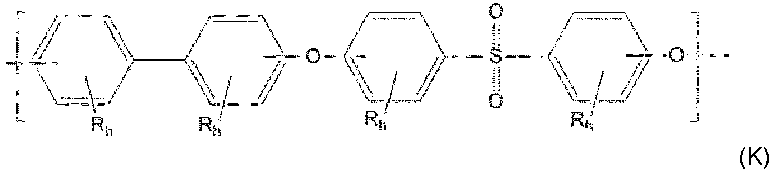 wobei
- R an jeder Stelle unabhängig ausgewählt ist aus einem Halogen, einem Alkyl, einem Alkenyl, einem Alkinyl, einem Aryl, einem Ether, einem Thioether, einer Carbonsäure, einem Ester, einem Amid, einem Imid, einem Alkali- oder Erdalkalimetallsulfonat, einem Alkylsulfonat, einem Alkali- oder Erdalkalimetallphosphonat, einem Alkylphosphonat, einem Amin und einem quaternären Ammonium; und
- h für jedes R unabhängig null oder eine ganze Zahl in dem Bereich von 1 bis 4 ist.

7. Verfahren gemäß einem der Ansprüche 1-5, wobei das Poly(biphenylethersulfon)-(Co)polymer (PPSU) wenigstens 60 mol-% Wiederholungseinheiten (RPPSU) der Formel (L) umfasst, wobei die mol-% auf die Gesamtzahl von Molen in dem Polymer bezogen sind:

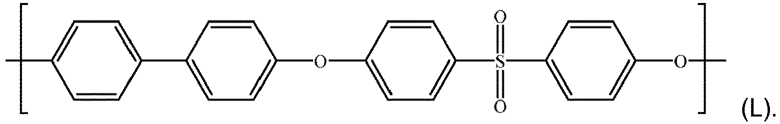

8. Verfahren gemäß Anspruch 6 oder 7, wobei das Poly(biphenylethersulfon)-(Co)polymer (PPSU) wenigstens 90 mol-% Wiederholungseinheiten (R_{PPSU}) der Formel (L) und/oder (K) umfasst, wobei die mol-% auf die Gesamtzahl von Molen in dem Polymer bezogen sind.

9. Filamentmaterial mit einer zylindrischen Geometrie und einem Durchmesser, der zwischen 0,5 und 5 mm ± 0,15 mm variiert, umfassend eine Polymerkomponente, die ein Poly(biphenylethersulfon)-(Co)polymer (PPSU) mit einem gewichtsgemittelten Molekulargewicht (Mw) in dem Bereich von 48.000 bis 52.000 g/mol (wie bestimmt durch Gelpermeationschromatographie (GPC) unter Verwendung von Methylenchlorid als mobile Phase und Polystyrolstandards) umfasst,
wobei das Poly(biphenylethersulfon)-(Co)polymer (PPSU) wenigstens 50 mol-% Wiederholungseinheiten (R_{PPSU}) der Formel (K) umfasst, wobei die mol-% auf die Gesamtzahl von Molen in dem Polymer bezogen sind: 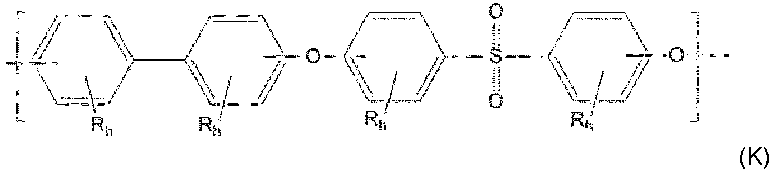 wobei
- R an jeder Stelle unabhängig ausgewählt ist aus einem Halogen, einem Alkyl, einem Alkenyl, einem Alkinyl, einem Aryl, einem Ether, einem Thioether, einer Carbonsäure, einem Ester, einem Amid, einem Imid, einem Alkali- oder Erdalkalimetallsulfonat, einem Alkylsulfonat, einem Alkali- oder Erdalkalimetallphosphonat, einem Alkylphosphonat, einem Amin und einem quaternären Ammonium; und
- h für jedes R unabhängig null oder eine ganze Zahl in dem Bereich von 1 bis 4 ist.

10. Filamentmaterial gemäß Anspruch 9, wobei die Polymerkomponente wenigstens 80 Gew.-% Poly(biphenylethersulfon)-(Co)polymer(e) (PPSU), bezogen auf das Gesamtgewicht der Polymerkomponente des Filamentmaterials, umfasst.

11. Verwendung eines Teilematerials, das eine Polymerkomponente umfasst, die wenigstens ein Poly(biphenylethersulfon)-(Co)polymer (PPSU) mit einem gewichtsgemittelten Molekulargewicht (Mw) in dem Bereich von 48.000 bis 52.000 g/mol (wie bestimmt durch Gelpermeationschromatographie (GPC) unter Verwendung von Methylenchlorid als mobile Phase und Polystyrolstandards) umfasst, für die Fertigung von dreidimensionalen Gegenständen unter Verwendung eines additiven Fertigungssystems,
wobei das Poly(biphenylethersulfon)-(Co)polymer (PPSU) wenigstens 50 mol-% Wiederholungseinheiten (R_{PPSU}) der Formel (K) umfasst, wobei die mol-% auf die Gesamtzahl von Molen in dem Polymer bezogen sind: 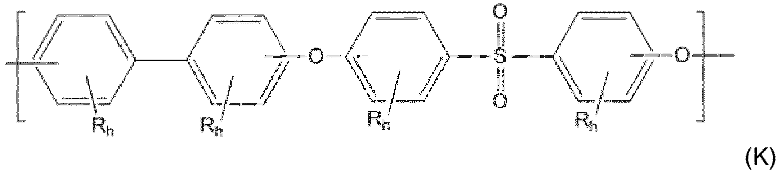 wobei
- R an jeder Stelle unabhängig ausgewählt ist aus einem Halogen, einem Alkyl, einem Alkenyl, einem Alkinyl, einem Aryl, einem Ether, einem Thioether, einer Carbonsäure, einem Ester, einem Amid, einem Imid, einem Alkali- oder Erdalkalimetallsulfonat, einem Alkylsulfonat, einem Alkali- oder Erdalkalimetallphosphonat, einem Alkylphosphonat, einem Amin und einem quaternären Ammonium; und
- h für jedes R unabhängig null oder eine ganze Zahl in dem Bereich von 1 bis 4 ist.

12. Verwendung gemäß Anspruch 11, wobei das Teilematerial ferner bis zu 30 Gew.-%, bezogen auf das Gesamtgewicht des Teilematerials, an wenigstens einem Zusatzstoff ausgewählt aus der Gruppe bestehend aus Füllstoffen, Farbstoffen, Schmiermitteln, Weichmachern, Flammhemmern, Keimbildnern, Fließverbesserern und Stabilisatoren umfasst.

13. Verwendung gemäß einem der Ansprüche 11-12, wobei die Polymerkomponente des Teilematerials wenigstens 80 Gew.-% Poly(biphenylethersulfon)-(Co)polymer(e) (PPSU), bezogen auf das Gesamtgewicht der Polymerkomponente des Teilematerials, umfasst.

14. Verwendung gemäß einem der Ansprüche 11-13, wobei das Teilematerial in der Form eines Filaments oder von Mikropartikeln vorliegt.

15. Verwendung eines Teilematerials, das eine Polymerkomponente umfasst, die wenigstens ein Poly(biphenylethersulfon)-(Co)polymer (PPSU) mit einem gewichtsgemittelten Molekulargewicht (Mw) in dem Bereich von 48.000 bis 52.000 g/mol (wie bestimmt durch Gelpermeationschromatographie (GPC) unter Verwendung von Methylenchlorid als mobile Phase und Polystyrolstandards) umfasst, für die Fertigung eines Filaments mit einer zylindrischen Geometrie und einem Durchmesser, der zwischen 0,5 und 5 mm ± 0,15 mm variiert, zur Verwendung bei der Fertigung von dreidimensionalen Gegenständen, wobei das Poly(biphenylethersulfon)-(Co)polymer (PPSU) wenigstens 50 mol-% Wiederholungseinheiten (R_{PPSU}) der Formel (K) umfasst, wobei die mol-% auf die Gesamtzahl von Molen in dem Polymer bezogen sind: 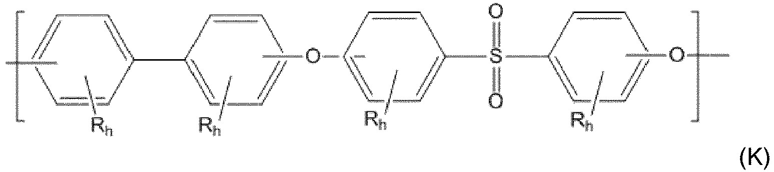 wobei
- R an jeder Stelle unabhängig ausgewählt ist aus einem Halogen, einem Alkyl, einem Alkenyl, einem Alkinyl, einem Aryl, einem Ether, einem Thioether, einer Carbonsäure, einem Ester, einem Amid, einem Imid, einem Alkali- oder Erdalkalimetallsulfonat, einem Alkylsulfonat, einem Alkali- oder Erdalkalimetallphosphonat, einem Alkylphosphonat, einem Amin und einem quaternären Ammonium; und
- h für jedes R unabhängig null oder eine ganze Zahl in dem Bereich von 1 bis 4 ist.

## Revendications

1. Procédé de fabrication d'un objet tridimensionnel (3D) à l'aide d'un système de fabrication additif, comprenant :
- la fourniture d'un matériau de pièce comprenant un constituant polymère comprenant au moins un (co)polymère de poly(biphényléthersulfone) (PPSU) ayant une masse moléculaire moyenne en poids (Mw) de 48 000 à 52 000 g/mol (telle que déterminée par chromatographie par perméation de gel (GPC) en utilisant du chlorure de méthylène comme phase mobile et des étalons en polystyrène),
- l'impression de couches de l'objet tridimensionnel à partir du matériau de pièce,
dans lequel le (co)polymère de poly(biphényléthersulfone) (PPSU) comprend au moins 50 %mol d'unités structurales (R_{PPSU}) selon la formule (K), le pourcentage molaire étant exprimé relativement au nombre total de moles dans le polymère : 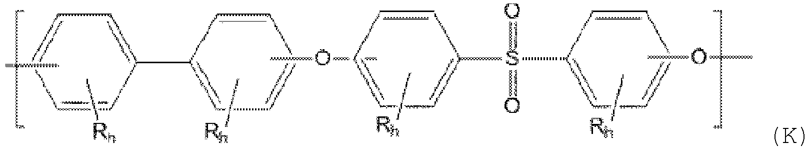 où
- R, à chaque emplacement, est sélectionné indépendamment parmi un groupe halogène, alkyle, alcényle, alkynyle, aryle, éther, thioéther, acide carboxylique, ester, amide, imide, sulfonate de métal alcalin ou alcalino-terreux, sulfonate d'alkyle, phosphonate de métal alcalin ou alcalino-terreux, phosphonate d'alkyle, amine, et ammonium quaternaire ; et
- h, pour chaque groupe R, est indépendamment zéro ou un nombre entier de 1 à 4.

2. Procédé selon la revendication 1, dans lequel le matériau de pièce comprend également jusqu'à 30 % en poids, relativement au poids total du matériau de pièce, d'au moins un additif sélectionné dans le groupe constitué de charges, de colorants, de lubrifiants, de plastifiants, de retardateurs de flamme, d'agents de nucléation, de fluidifiants et de stabilisants.

3. Procédé selon la revendication 1 ou 2, dans lequel le constituant polymère du matériau de pièce comprend au moins 80 % en poids de (co)polymère(s) de poly(biphényléthersulfone) (PPSU), relativement au poids total de constituant polymère du matériau de pièce.

4. Procédé selon l'une quelconque des revendications 1 à 3, dans lequel le matériau de pièce est sous la forme d'un filament ou de microparticules.

5. Procédé selon l'une quelconque des revendications 1 à 4, dans lequel l'étape d'impression de couches comprend l'extrusion du matériau de pièce.

6. Procédé selon l'une quelconque des revendications 1 à 5, dans lequel le (co)polymère de poly(biphényléthersulfone) (PPSU) comprend au moins 60 %mol d'unités structurales (R_{PPSU}) selon la formule (K), le pourcentage molaire étant exprimé relativement au nombre total de moles dans le polymère : 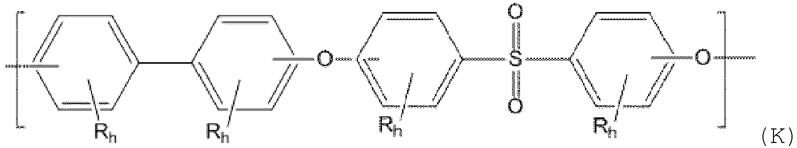 où
- R, à chaque emplacement, est sélectionné indépendamment parmi un groupe halogène, alkyle, alcényle, alkynyle, aryle, éther, thioéther, acide carboxylique, ester, amide, imide, sulfonate de métal alcalin ou alcalino-terreux, sulfonate d'alkyle, phosphonate de métal alcalin ou alcalino-terreux, phosphonate d'alkyle, amine, et ammonium quaternaire ; et
- h, pour chaque groupe R, est indépendamment zéro ou un nombre entier de 1 à 4.

7. Procédé selon l'une quelconque des revendications 1 à 5, dans lequel le (co)polymère de poly(biphényléthersulfone) (PPSU) comprend au moins 60 %mol d'unités structurales (R_{PPSU}) selon la formule (L), le pourcentage molaire étant exprimé relativement au nombre total de moles dans le polymère :

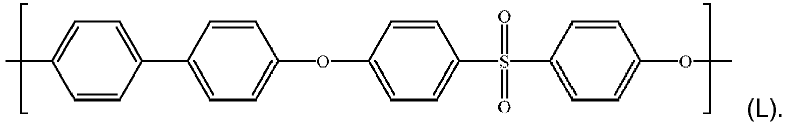

8. Procédé selon la revendication 6 ou 7, dans lequel le (co)polymère de poly(biphényléthersulfone) (PPSU) comprend au moins 90 %mol d'unités structurales (R_{PPSU}) selon la formule (L) et/ou (K), le pourcentage molaire étant exprimé relativement au nombre total de moles dans le polymère.

9. Matériau sous forme de filament ayant une géométrie cylindrique et un diamètre de 0,5 à 5 mm ± 0,15 mm, comprenant un constituant polymère comprenant un (co)polymère de poly(biphényléthersulfone) (PPSU) ayant une masse moléculaire moyenne en poids (Mw) de 48 000 à 52 000 g/mol (telle que déterminée par chromatographie par perméation de gel (GPC) en utilisant du chlorure de méthylène comme phase mobile et des étalons en polystyrène),
dans lequel le (co)polymère de poly(biphényléthersulfone) (PPSU) comprend au moins 50 %mol d'unités structurales (R_{PPSU}) selon la formule (K), le pourcentage molaire étant exprimé relativement au nombre total de moles dans le polymère : 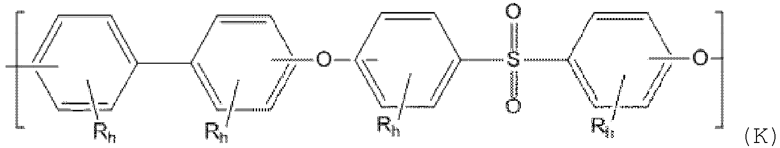 où
- R, à chaque emplacement, est sélectionné indépendamment parmi un groupe halogène, alkyle, alcényle, alkynyle, aryle, éther, thioéther, acide carboxylique, ester, amide, imide, sulfonate de métal alcalin ou alcalino-terreux, sulfonate d'alkyle, phosphonate de métal alcalin ou alcalino-terreux, phosphonate d'alkyle, amine, et ammonium quaternaire ; et
- h, pour chaque groupe R, est indépendamment zéro ou un nombre entier de 1 à 4.

10. Matériau sous forme de filament selon la revendication 9, dans lequel le constituant polymère comprend au moins 80 % en poids de (co)polymère de poly(biphényléthersulfone) (PPSU), relativement au poids total du constituant polymère du matériau sous forme de filament.

11. Utilisation d'un matériau de pièce comprenant un constituant polymère comprenant au moins un (co)polymère de poly(biphényléthersulfone) (PPSU) ayant une masse moléculaire moyenne en poids (Mw) de 48 000 à 52 000 g/mol (telle que déterminée par chromatographie par perméation de gel (GPC) en utilisant du chlorure de méthylène comme phase mobile et des étalons en polystyrène), pour la fabrication d'objets tridimensionnels à l'aide d'un système de fabrication additif,
dans laquelle le (co)polymère de poly(biphényléthersulfone) (PPSU) comprend au moins 50 %mol d'unités structurales (R_{PPSU}) selon la formule (K), le pourcentage molaire étant exprimé relativement au nombre total de moles dans le polymère : 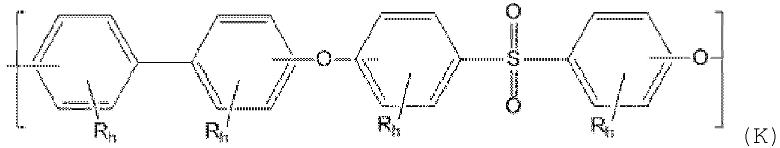 où
- R, à chaque emplacement, est sélectionné indépendamment parmi un groupe halogène, alkyle, alcényle, alkynyle, aryle, éther, thioéther, acide carboxylique, ester, amide, imide, sulfonate de métal alcalin ou alcalino-terreux, sulfonate d'alkyle, phosphonate de métal alcalin ou alcalino-terreux, phosphonate d'alkyle, amine, et ammonium quaternaire ; et
- h, pour chaque groupe R, est indépendamment zéro ou un nombre entier de 1 à 4.

12. Utilisation selon la revendication 11, dans laquelle le matériau de pièce comprend également jusqu'à 30 % en poids, relativement au poids total du matériau de pièce, d'au moins un additif sélectionné dans le groupe constitué de charges, de colorants, de lubrifiants, de plastifiants, de retardateurs de flamme, d'agents de nucléation, de fluidifiants et de stabilisants.

13. Utilisation selon l'une quelconque des revendications 11 ou 12, dans laquelle le constituant polymère du matériau de pièce comprend au moins 80 % en poids de (co)polymère de poly(biphényléthersulfone) (PPSU), relativement au poids total de constituant polymère du matériau de pièce.

14. Utilisation selon l'une quelconque des revendications 11 à 13, dans laquelle le matériau de pièce est sous la forme d'un filament ou de microparticules.

15. Utilisation d'un matériau de pièce comprenant un constituant polymère comprenant au moins un (co)polymère de poly(biphényléthersulfone) (PPSU) ayant une masse moléculaire moyenne en poids (Mw) de 48 000 à 52 000 g/mol (telle que déterminée par chromatographie par perméation de gel (GPC) en utilisant du chlorure de méthylène comme phase mobile et des étalons en polystyrène), pour la fabrication d'un filament ayant une géométrie cylindrique et un diamètre de 0,5 à 5 mm ± 0,15 mm, destiné à une utilisation dans la fabrication d'objets tridimensionnels,
dans laquelle le (co)polymère de poly(biphényléthersulfone) (PPSU) comprend au moins 50 %mol d'unités structurales (R_{PPSU}) selon la formule (K), le pourcentage molaire étant exprimé relativement au nombre total de moles dans le polymère : 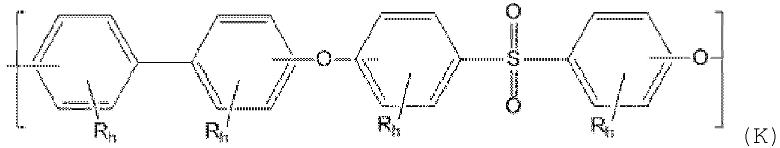 où
- R, à chaque emplacement, est sélectionné indépendamment parmi un groupe halogène, alkyle, alcényle, alkynyle, aryle, éther, thioéther, acide carboxylique, ester, amide, imide, sulfonate de métal alcalin ou alcalino-terreux, sulfonate d'alkyle, phosphonate de métal alcalin ou alcalino-terreux, phosphonate d'alkyle, amine, et ammonium quaternaire ; et
- h, pour chaque groupe R, est indépendamment zéro ou un nombre entier de 1 à 4.
